(19) Europäisches Patentamt
European Patent Office
Office européen des brevets

(11) **EP 4 700 383 A1**

(12) **EUROPEAN PATENT APPLICATION**

(43) Date of publication:
**25.02.2026 Bulletin 2026/09**

(21) Application number: **24195743.0**

(22) Date of filing: **21.08.2024**

(51) International Patent Classification (IPC):
**G01N 27/12** (2006.01)

(52) Cooperative Patent Classification (CPC):
**G01N 27/128; G01N 25/18; G01N 33/005**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Tempus Inc.**
**Seoul 01811 (KR)**

(72) Inventor: **LEE, Byoung Su**
**01816 Seoul (KR)**

(74) Representative: **Marks & Clerk LLP**
**15 Fetter Lane**
**London EC4A 1BW (GB)**

(54) **HYDROGEN AND HELIUM GAS SENSOR**

(57)     A hydrogen and helium gas sensor according to an embodiment may include a substrate having a through-hole; a diaphragm including a first diaphragm formed to protrude toward a portion where the through-hole is formed, and a second diaphragm formed at an opposing portion of the first diaphragm to be separated from the first diaphragm; a heater formed on one side of the first diaphragm; a first sensor formed on another side of the first diaphragm to measure an amount of conduc-tion heat transferred from the heater through the first diaphragm by a conduction method; a second sensor formed on the second diaphragm to measure an amount of convection heat transferred through air in the through-hole from the heater by a convection method; a gas concentration calculating unit for calculating a concentration of hydrogen gas or helium gas by using the first sensor and the second sensor.

FIG. 1

## Description

### BACKGROUND

1. Field of the Disclosure

The present disclosure relates to a gas sensor, and more specifically to a hydrogen and helium gas sensor.

2. Description of the Related Art

Hydrogen is a raw material for a fuel cell and is a gas that leaks out during charging/discharging and malfunction of a lithium-ion battery, and measuring hydrogen concentration is an important technique for preventing battery explosion and monitoring battery failure.

An electrochemical sensor, which is operated on a same principle as a metal oxide semiconductor (MOS) or fuel cell, is widely used to measure hydrogen concentration. However, there is a limitation that these methods should be used in absence of other gases due to a cross-sensing phenomenon in which the sensor reacts to other combustion gases (e.g., methane ($CH_4$), ethanol ($C_2H_6O$), carbon monoxide (CO), etc.).

A method of solving this disadvantage is to use a convection gauge as a method of using characteristics that the heat transfer rate of light gases such as hydrogen or helium is very high compared to that of other types of heavy gases. Generally, compared to nitrogen gas (N2) or oxygen gas (O2), helium gas has a heat transfer rate of about 6 times at room temperature, and hydrogen gas has a heat transfer rate of about 7 times.

Gas measurement using a conventional convection uses a structure in which a heater and a thermal sensor are disposed on top of a structure. In this case, concentration of hydrogen and helium gas with a high heat transfer rate is estimated by checking how much heat applied to the heater is transferred to the thermal sensor. However, since a method of maintaining a constant temperature by applying a constant current to the heater and measuring the heat transferred to the thermal sensor is used, the resolution is low, therefore, there is a disadvantage that only the concentration of hydrogen or helium gas with a high concentration of 1% or more can be measured.

### SUMMARY

The present disclosure is intended to solve the above-mentioned problem and is intended to provide a gas sensor configured to increase resolution of concentration measurement in a hydrogen and helium gas sensor using heat transfer by convection. However, this problem is exemplary and the scope of the present disclosure is not limited thereto.

According to an aspect of the present disclosure, a hydrogen and helium gas sensor may include: a substrate having a through-hole; a diaphragm including a first diaphragm formed on the substrate and formed to protrude toward a portion where the through-hole is formed and a second diaphragm formed at a portion facing the first diaphragm to be spaced apart from the first diaphragm by a predetermined distance; a heater formed on one side of the first diaphragm and transferring heat to surroundings by generating heat by a supplied current; a first sensor formed on the other side of the first diaphragm and measuring an amount of conduction heat transferred from the heater through the first diaphragm by conduction; a second sensor formed on the second diaphragm and measuring an amount of convection heat transferred through air in the through-hole from the heater by convection; and a gas concentration calculating unit for calculating a concentration of hydrogen gas or helium gas contained in the air in the through-hole by using a first signal for the amount of conduction heat measured from the first sensor and a second signal for the amount of convection heat measured from the second sensor.

According to the hydrogen and helium gas sensor, the gas concentration calculating unit may calculate the concentration of hydrogen gas or helium gas by comparing the second signal with a reference signal set for reference air.

According to the hydrogen and helium gas sensor, the gas concentration calculating unit may calculate the concentration of hydrogen gas or helium gas by comparing the second signal with a reference signal set for reference air under a reference heat generation amount when the first signal is within a reference range.

According to the hydrogen and helium gas sensor, the gas concentration calculating unit may calculate a heat generation amount of the heater through the first signal, correct the second signal according to the heat generation amount of the heater, and calculate the concentration of hydrogen gas or helium gas by comparing the second signal with a reference signal set for reference air under a reference heat generation amount.

According to the hydrogen and helium gas sensor, the gas concentration calculating unit may correct the second signal when the first signal is outside a reference range, and may not correct the second signal when the first signal is within the reference range.

According to the hydrogen and helium gas sensor, the gas concentration calculating unit may calculate that the amount of hydrogen gas or helium gas in the air in the through-hole increases as the second signal increases.

According to the hydrogen and helium gas sensor, the reference air may be normal atmospheric air containing nitrogen, oxygen, and argon, and the through-hole may be filled with air to be measured.

According to the hydrogen and helium gas sensor, the first diaphragm and the second diaphragm may be disposed on a same level line.

According to the hydrogen and helium gas sensor, the first diaphragm may be formed to be longer than the second diaphragm.

BRIEF DESCRIPTION OF THE DRAWINGS

FIG. 1 is a perspective view schematically showing a structure of a hydrogen and helium gas sensor according to an embodiment of the present disclosure.

FIG. 2 is a cross-sectional view of a sensor taken along line AA shown in FIG. 1.

FIG. 3 is a voltage signal measured at a first sensor, which measures an amount of conduction heat when a periodic pulse is applied to a heater of a hydrogen and helium gas sensor according to an embodiment of the present disclosure.

FIG. 4 is a graph showing a relationship between a first signal and a second signal according to hydrogen concentration.

DETAILED DESCRIPTION OF THE EMBODIMENTS

Hereinafter, various preferred embodiments of the present disclosure will be described in detail with reference to the attached drawings.

Various embodiments of the present disclosure may be embodied in many different forms and should not be construed as being limited to the example embodiments set forth herein. Rather, these example embodiments of the disclosure are provided so that this disclosure will be thorough and complete and will convey inventive concepts of the disclosure to those skilled in the art.

Throughout the drawings, for example, according to manufacturing technologies and/or tolerances, illustrated shapes may be modified. Also, in the drawings, the thicknesses or sizes of layers are exaggerated for clarity. Thus, the embodiments of the present disclosure should not be construed as limited to the particular shapes of regions illustrated herein, but are to include deviations in shapes that result, for example, from manufacturing.

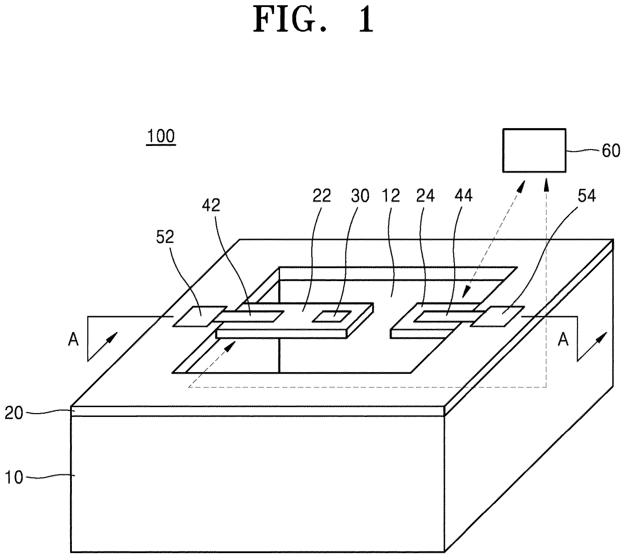

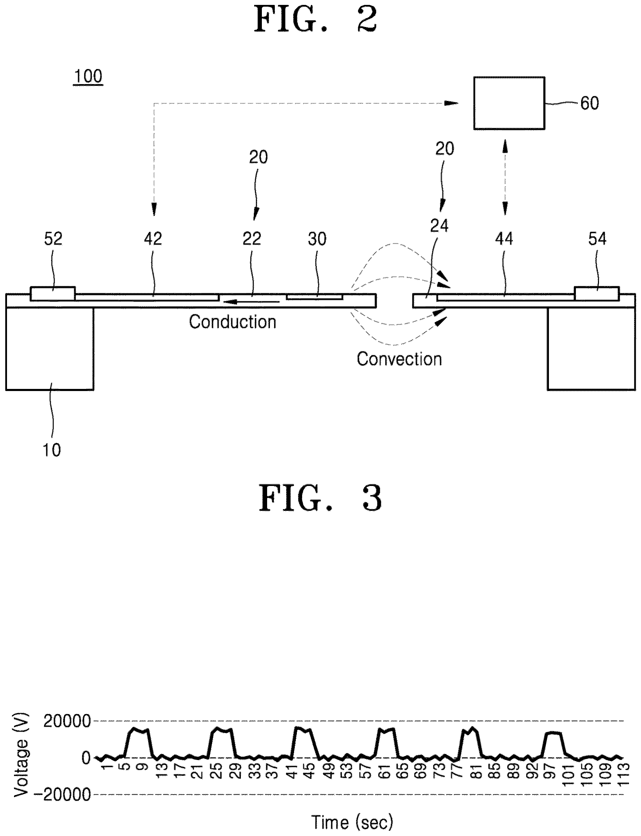

FIG. 1 is a perspective view schematically showing a structure of a hydrogen and helium gas sensor according to an embodiment of the present disclosure, and FIG. 2 is a cross-sectional view of a sensor taken along line AA shown in FIG. 1.

Referring to FIGS. 1 and 2, the hydrogen and helium gas sensor 100 according to an embodiment of the present disclosure may include a substrate 10, a diaphragm 20, a heater 30, a first sensor 42, and a second sensor 44.

For example, the hydrogen and helium gas sensor 100 using convection includes the substrate 10 having a through-hole 12, and the diaphragm 20 formed on the substrate 10. The substrate 10, for example, may be a semiconductor substrate including silicon (Si). The diaphragm 20 includes a first diaphragm 22 and a second diaphragm 24 and may be formed using a micro-electromechanical systems (MEMS) process.

The first diaphragm 22 and the second diaphragm 24 may be formed to protrude to an inner portion where the through-hole 12 provided on the substrate 10 is formed. Here, the first diaphragm 22 and the second diaphragm 24 are formed on portions facing each other and may be physically separated from each other to be spaced apart by a predetermined distance.

In some embodiments, the first diaphragm 22 and the second diaphragm 24 may be formed on a same level line. For example, the first diaphragm 22 and the second diaphragm 24 may be formed to protrude toward a direction in which the through-hole 12 provided on the substrate 10 is formed to form a bridge structure cut in the through-hole 12. The first diaphragm 22 and the second diaphragm 24 are formed to allow air flow in an empty space formed by cutting based on a certain portion.

The first diaphragm 22 and the second diaphragm 24 have different functions, and lengths of the first diaphragm 22 and the second diaphragm 24 are formed to be different from each other. For example, the heater 30 may be formed on the first diaphragm 22, and accordingly, the length of the first diaphragm 22 may be formed to be relatively longer than the length of the second diaphragm 24. The first diaphragm 22 may be formed of a heat transfer material capable of heat conduction, and further, the second diaphragm 24 may also be formed of the heat transfer material. The heater 30 is formed on one side of the first diaphragm 22, and transfers heat to the surroundings by a current supplied to the heater 30. The heat generated from the heater 30 may be transferred to the first sensor 42 and the second sensor 44. The first sensor 42 and the second sensor 44 may include various heat sensors capable of measuring heat. For example, the first sensor 42 and the second sensor 44 may use a thermopile sensor (sensors), a bolometer, or a pyroelectric sensor (sensors).

The first sensor 42 is formed on the other side of the first diaphragm 22 and may be provided to measure the amount of conduction heat transferred by conduction from the heater 30 through the first diaphragm 22. In this respect, the first sensor 42 may be referred to as a conduction sensor. The heater 30 and the first sensor 42 may be formed on both sides of the first diaphragm 22 to face each other. For example, the heater 30 may be formed on a center-side end of the through-hole 12 of the first diaphragm 22 facing the second diaphragm 24, and the first sensor 42 may be formed on an edge-end of the through-hole 12 of the first diaphragm 22 to be spaced apart from the heater 30 by a predetermined distance.

The second sensor 44 is formed on the second diaphragm 24 and may be provided to measure the amount of conduction heat transferred through the air in the through-hole 12 by convection from the heater 30. In this respect, the second sensor 44 may be referred to as the convection sensor.

When the heater 30 is formed on the first diaphragm 22 near the center of the through-hole 12, the first sensor 42 and the second sensor 44 may be disposed on opposite sides of each other with respect to the heater 30. Accordingly, the heat of the heater 30 may be transferred to the first sensor 42 by conduction through the first diaphragm 22, and to the second sensor 24 by convection through the air in a cut part between the first diaphragm 22 and the second diaphragm 24.

As described above, the hydrogen and helium gas sensor 100 heater 30 includes one heater 30 formed at a center of the diaphragm 20, and the first and the second sensors 42, 44 disposed in parallel at both ends of the heater 30. Among them, the first sensor 42 has a structure thermally connected to the heater 30 through the first diaphragm 22. The second sensor 44 is not connected through the heater 30 and the first diaphragm 22 and has a structure formed on the second diaphragm 24 to enable heat transfer only through convection by air.

In some embodiments, the first sensor 42 may be fixed by a first fixing part 52 on the substrate 10. Furthermore, the second sensor 44 may be fixed by a second fixing part 54 on the substrate 10.

In some embodiments, the hydrogen and helium gas sensor 100 may further include a gas concentration calculating unit 60. The gas concentration calculating unit 60 may transmit and receive signals to and from the first sensor 42 and the second sensor 44 and may calculate a concentration of hydrogen and/or helium based on this. For example, the gas concentration calculating unit 60 may calculate the concentration of hydrogen gas and/or helium gas contained in the air in the through-hole 12 through a first signal for the amount of conduction heat measured from the first sensor 42 and a second signal for an amount of convection heat measured from the second sensor 44. Therefore, when measuring the concentration, air to be measured is first filled in the air in the through-hole 12, and then the signals of the first sensor 42 and the second sensor 44 may be measured after heating the heater 30.

The gas concentration calculating unit 60 may basically calculate the concentration of hydrogen gas and/or helium gas based on the second signal measured from the second sensor 44 which is the convection sensor, and the first signal measured from the first sensor 42 which is the conduction sensor may be used to correct the second signal. In this respect, the hydrogen and helium gas sensor 100 according to the embodiments may also be referred to as a convection gas sensor.

In some embodiments, the gas concentration calculating unit 60 may calculate the concentration of hydrogen gas or helium gas by comparing the second signal with a reference signal set for reference air. Since the heat transfer rate of light gases such as hydrogen or helium has a very high convection heat transfer rate compared to that of other types of heavy gases, as the concentration of hydrogen or helium gas in the air in the through-hole 12 increases, the heat transfer rate using convection increases, thereby increasing the magnitude of the second signal measured from the second sensor 44. Therefore, the gas concentration calculating unit 60 may calculate the concentration of hydrogen gas and/or helium gas in the air in through-hole 12 by comparing the second signal measured in the second sensor 44 with the reference signal. Furthermore, the gas concentration calculating unit 60 may calculate that the amount of hydrogen gas or helium gas in the air in the through-hole 12 increases as the second signal increases.

In some embodiments, the reference signal may be predetermined air with a defined composition, for example, normal atmospheric air. For example, the atmospheric air contains nitrogen, oxygen, and argon, and concentration of hydrogen and helium may be extremely low. Therefore, normal air containing very little hydrogen and helium gas is filled in the through-hole 12 of the hydrogen and helium gas sensor 100, the second signal is measured through the second sensor 44, and the signal may be set as the reference signal.

In some embodiments, when the first signal is within a reference range, the gas concentration calculating unit 60 may calculate the concentration of hydrogen gas or helium gas by comparing the second signal with the reference signal set for the reference air under a reference heat generation amount. When the heat generation amount generated from the heater 30 changes, the second signal may be changed regardless of the concentration of hydrogen and/or helium gas in the air. Therefore, when the heat generation amount of the heater 30 is within a certain range, that is, when the first signal is within a reference range, the concentration of hydrogen gas or helium gas may be calculated by comparing the second signal with the reference signal.

In some embodiments, the gas concentration calculating unit 60 may calculate the heat generation amount of the heater 30 through the first signal, correct the second signal according to the heat generation amount of the heater 30, and compare the corrected second signal with the reference signal set for the reference air under the reference heat generation amount to calculate the concentration of hydrogen gas or helium gas. As described above, when the heat generation amount of heater 30 changes, it is necessary to correct the second signal accordingly. When the heat generation amount of the heater 30 becomes larger, the second signal may also increase proportionally, and thus the second signal may be corrected by converting into it in case of the reference heat generation amount. For example, the reference heat generation amount may be set to a certain value in consideration of a voltage and a current applied to the heater 30.

Optionally, the gas concentration calculating unit 60 may correct the second signal when the first signal is outside the reference range, and may not correct the second signal when the first signal is within the reference range.

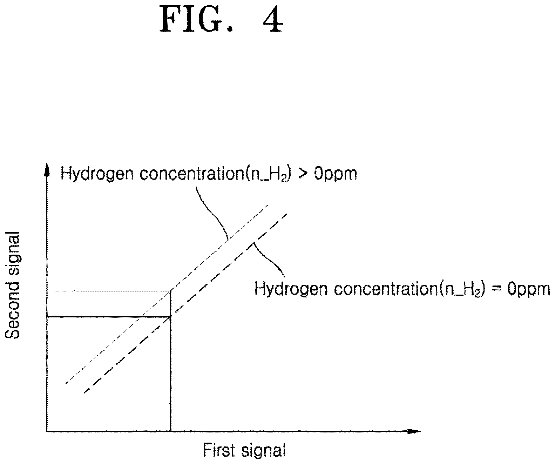

Hereinafter, a method of measuring the concentration of hydrogen and helium gas using a gas concentration calculating unit (not shown) will be described in more detail with reference to FIGS. 3 and 4.

FIG. 3 is a voltage signal generated at a first sensor, which measures an amount of conduction heat when a periodic pulse is applied to a heater of a hydrogen and helium gas sensor 100 according to an embodiment of the present disclosure, and FIG. 4 is a graph showing a relationship between a first signal and a second signal according to hydrogen concentration.

In general, the heat generated by power applied to the heater 30 changes depending on resistance of the heater 30 and current applied to the heater 30. In order to accurately measure the heat generated from the heater 30, there is a method of measuring the current and voltage of the heater 30. However, there is a problem in accurately specifying due to an environmental change such as external humidity and a change due to aging of a state of a resistance thin film of the heater 30.

In order to solve this problem, the present disclosure attempted to accurately monitor a heat generation amount of the heater 30 using the first sensor. For example, by forming the heater 30 at one end of the first diaphragm 22 and forming the first sensor 42 at the other end, the heat generated from the heater 30 may be directly transferred to the first sensor 42 through the first diaphragm 22, and thus heating status of the heater 30 may be directly monitored. Further, the second sensor 44 is disposed adjacent to the heater 30 and detects the heat signal caused by convention by receiving the heat through the air using a convection phenomenon, and thus may measure the amount of convection heat.

When assuming that the heat transfer rate of air (composed of nitrogen (N), oxygen (O), and argon (Ar), etc.) at constant atmospheric pressure is 1, the heat transfer rate of a gas with a high heat transfer rate, such as hydrogen gas ($H_2$), is defined as t_H, and the concentration of the gas with a high heat transfer rate is defined as n (however, $0 < n < 1$), the heat transfer rate of a mixture of general air and hydrogen having different composition ratios from each other may be expressed as Equation 1 below. Here, the gas having a high heat transfer rate refers to a gas having a relatively higher heat transfer rate than that of air.

[Equation 1]

$$t\_mix = (1\text{-}n) + t\_H \times n = 1 + n \times (t\_H - 1)$$

(The t_mix refers to a heat transfer rate of the entire air containing gas whose heat transfer rate is relatively higher than that of air, n refers to a concentration of gas whose heat transfer rate is relatively higher than that of air, and t_H refers to a heat transfer rate of gas whose heat transfer rate is relatively higher than that of air)

As an example, when hydrogen gas is contained in the air, according to the Equation 1, the heat transfer rate by the hydrogen gas may be expressed as Equation 2 below.

[Equation 2]

$$\text{Heat transfer rate of hydrogen gas } (t\_H) = 1 + 6 \times n\_H_2$$

(the n_$H_2$ refers to the concentration of the hydrogen gas)

As another example, when helium gas is contained in the air, according to the Equation 1, the heat transfer rate by the helium gas may be expressed as Equation 3 below.

[Equation 3]

$$\text{Heat transfer rate of helium gas } (t\_He) = 1 + 5 \times n\_He$$

(the n_He refers to the concentration of helium gas)

According to the Equation 1, depending on whether a gas with a relatively higher heat transfer rate than that of air is contained, a difference between the heat transfer rate in a general air state and the measured heat transfer rate is all proportional to the concentration of hydrogen gas or helium gas as shown in FIG. 4. Using this, it is possible to accurately calculate the concentration of hydrogen gas or helium gas through the heat transfer rate in the gas concentration calculating unit 60.

Heat is generated by applying a current to the heater 30, the heat is transferred to the first sensor 42 by conduction, and the heat generation amount of the heater 30 may be measured by detecting the generated heat signal in the first sensor 42. For example, as shown in FIG. 3, it can be confirmed that a voltage signal generated at the first sensor 42 is measured consistently when a periodic pulse is applied to the heater 30. As such, the gas concentration calculating unit 60 may process a function of confirming whether the voltage signal measured by the first sensor 42 is constant and detecting an abnormality due to instability of a power such as the voltage or current applied to the heater 30.

Further, the gas concentration calculating unit 60 may detect the heat signal generated from the heater 30 transmitted to the second sensor 44 using the convection phenomenon. In this case, when there is no change in the type of gas contained in the air, the heat signal detected in the first sensor 42 and the second sensor 44 may always remain constant.

When the air contains a gas having a higher heat transfer rate than that of the air, the heat signal detected at the second sensor 44 may increase when the heat signal detected at the first sensor 42 is constant.

Referring to FIG. 4, in a case where concentration of highly conductive gas is 0, and when a signal value of the first sensor 42 changes without a change in air composition, this is caused by a change in an electrical factor applied to the heater 30 and thus the signal of the second sensor 44 is also changed proportionally so that a line may be drawn on a phase space for n=0. Further, even in a case of n>0, since a line appears on the phase space, it is possible to estimate to which line the measured signal of first sensor 42 and second sensor 44 are close to.

By using the hydrogen and helium gas sensor 100 using convection according to an embodiment of the present disclosure, when air contains a gas with a higher heat transfer rate than the air, it may be effectively detected even if the concentration of the gas with a high heat transfer rate contained in the air is slightly low by the above-described manner.

According to the hydrogen and helium gas sensor according to an embodiment of the present disclosure as described above, by calculating the heat generation amount of the heater with high accuracy through the first sensor that measures the amount of conduction heat, whether the heat generation amount changes according to the change in power applied to the heater is determined, and by correcting the signal of the second sensor according to the change in the heat generation amount, the resolution of measuring the concentration of hydrogen and helium can be increased. However, the scope of the present disclosure is not limited by these effects.

The present disclosure has been described with reference to the embodiments illustrated in the drawings, but these embodiments are merely illustrative and it should be understood by a person with ordinary skill in the art that various modifications and equivalent embodiments may be made without departing from the scope of the present disclosure. Therefore, the true technical protective scope of the present disclosure should be determined based on the technical concept of the appended claims.

**Claims**

1. A hydrogen and helium gas sensor comprising:

   a substrate having a through-hole;
   a diaphragm including a first diaphragm formed on the substrate and formed to protrude toward a portion where the through-hole is formed, and a second diaphragm formed on a portion facing the first diaphragm to be spaced apart from the first diaphragm by a predetermined distance;
   a heater formed on one side of the first diaphragm and transferring heat to surroundings by generating heat by a supplied current;
   a first sensor formed on the other side of the first diaphragm and measuring an amount of conduction heat transferred from the heater through the first diaphragm by conduction;
   a second sensor formed on the second diaphragm and measuring an amount of convection heat transferred through air in the through-hole from the heater by convection; and
   a gas concentration calculating unit for calculating a concentration of hydrogen gas or helium gas in the air in the through-hole by using a first signal for the amount of conduction heat measured from the first sensor and a second signal for the amount of convection heat measured from the second sensor.

2. The hydrogen and helium gas sensor of claim 1, wherein the gas concentration calculating unit calculates the concentration of hydrogen gas or helium gas by comparing the second signal with a reference signal set for reference air.

3. The hydrogen and helium gas sensor of claim 1, wherein the gas concentration calculating unit calculates the concentration of hydrogen gas or helium gas by comparing the second signal with a reference signal set for reference air under a reference heat generation amount when the first signal is within a reference range.

4. The hydrogen and helium gas sensor of claim 1, wherein the gas concentration calculating unit calculates a heat generation amount of the heater through the first signal, corrects the second signal according to the heat generation amount of the heater, and calculates the concentration of hydrogen gas or helium gas by comparing the second signal with a reference signal set for reference air under a reference heat generation amount.

5. The hydrogen and helium gas sensor of claim 4, wherein the gas concentration calculating unit corrects the second signal when the first signal is outside a reference range, and does not correct the second signal when the first signal is within the reference range.

6. The hydrogen and helium gas sensor of claim 3, wherein the gas concentration calculating unit calculates that the amount of hydrogen gas or helium gas in the air in the through-hole increases as the second signal increases.

7. The hydrogen and helium gas sensor of claim 2, wherein the reference air is normal atmospheric air containing nitrogen, oxygen and argon, and the through-hole is filled with air to be measured.

8. The hydrogen and helium gas sensor of claim 1, wherein the first diaphragm and the second diaphragm are disposed on a same level line.

9. The hydrogen and helium gas sensor of claim 1, wherein the first diaphragm is formed to be longer than the second diaphragm.

# FIG. 1

# FIG. 2

# FIG. 3

# FIG. 4

Hydrogen concentration(n_H$_2$) > 0ppm

Hydrogen concentration(n_H$_2$) = 0ppm

Second signal

First signal

Europäisches
Patentamt

European
Patent Office

Office européen
des brevets

## EUROPEAN SEARCH REPORT

**Application Number**

EP 24 19 5743

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/268892 A2 (FLUSSO LTD [GB]) 29 December 2022 (2022-12-29) * figures 1-48 * * page 2, line 13 - page 58, line 5 * ----- | 1-9 | INV. G01N27/12 |
| X | US 2023/194450 A1 (RÜEGG ANDREAS [CH] ET AL) 22 June 2023 (2023-06-22) * figures 1-9 * * paragraph [0001] - paragraph [0147] * ----- | 1-8 | |
| A | KR 102 625 936 B1 (INNER SENSOR CO LTD [KR]) 17 January 2024 (2024-01-17) * the whole document * ----- | 1-9 | |

| | |
|---|---|
| | **TECHNICAL FIELDS SEARCHED (IPC)** |
| | G01N |

The present search report has been drawn up for all claims

| Place of search | Date of completion of the search | Examiner |
|---|---|---|
| Munich | 24 January 2025 | Colasanti, Katharina |

EPO FORM 1503 03.82 (P04C01)

## ANNEX TO THE EUROPEAN SEARCH REPORT
## ON EUROPEAN PATENT APPLICATION NO.

EP 24 19 5743

This annex lists the patent family members relating to the patent documents cited in the above-mentioned European search report.
The members are as contained in the European Patent Office EDP file on
The European Patent Office is in no way liable for these particulars which are merely given for the purpose of information.

24-01-2025

| Patent document cited in search report | | Publication date | Patent family member(s) | | Publication date |
|---|---|---|---|---|---|
| WO 2022268892 | A2 | 29-12-2022 | CN | 117597580 A | 23-02-2024 |
| | | | EP | 4359777 A2 | 01-05-2024 |
| | | | WO | 2022268892 A2 | 29-12-2022 |
| US 2023194450 | A1 | 22-06-2023 | CN | 116265926 A | 20-06-2023 |
| | | | EP | 4198504 A1 | 21-06-2023 |
| | | | US | 2023194450 A1 | 22-06-2023 |
| KR 102625936 | B1 | 17-01-2024 | KR | 102625936 B1 | 17-01-2024 |
| | | | WO | 2024190955 A1 | 19-09-2024 |

EPO FORM P0459

For more details about this annex : see Official Journal of the European Patent Office, No. 12/82